# EUROPEAN PATENT SPECIFICATION

(11) **EP 2 686 004 B1**
(45) Date of publication and mention of the grant of the patent: **12.09.2018**
(21) Application number: 12711009.6
(22) Date of filing: 16.03.2012
(51) Int. Cl.: A23L 33/10, A23L 33/17, A23L 33/105, A61K 38/44, A61K 9/107, A61K 9/48, A61K 9/70, C12N 9/00, A61P 21/02, A61P 25/06, A23P 10/30, A23P 10/40

(54) **COMPOSITION COMPRISING DIAMINE OXIDASE FOR USE IN THE TREATMENT OR PREVENTION OF FIBROMYALGIA OR CHRONIC FATIGUE SYNDROME**
ZUSAMMENSETZUNG MIT DIAMINOXIDASE ZUR VERWENDUNG BEI DER BEHANDLUNG ODER VORBEUGUNG VON FIBROMYALGIE ODER CHRONISCHEM MÜDIGKEITSSYNDROM
COMPOSITION COMPRENANT UNE DIAMINE OXYDASE POUVANT ÊTRE UTILISÉE POUR TRAITER OU PRÉVENIR LA FIBROMYALGIE OU LE SYNDROME DE FATIGUE CHRONIQUE

(30) Priority: 18.03.2011 ES 201130383 P
(43) Date of publication of application: 22.01.2014
(73) Proprietor: Dr Healthcare España, S. L., 08017 Barcelona (ES)
(72) Inventor: DUELO RIU, Carlos, 08017 Barcelona (ES); DUELO RIU, Juan José, 08017 Barcelona (ES)
(74) Representative: Ponti & Partners, S.L.P
(86) International application number: PCT/IB2012/051276
(87) International publication number: WO 2012/127392

(56) References cited:
- EP-A2- 0 132 674
- EP-A2- 1 277 477
- WO-A2-02/43745
- WO-A2-03/035000
- FR-A1- 2 101 095
- FR-A1- 2 215 944
- GB-A- 1 313 318
- US-A- 3 721 733
- US-A- 4 725 540
- US-A1- 2008 193 491
- US-A1- 2008 227 116
- MAINTZ LAURA ET AL: "Histamine and histamine intolerance", THE AMERICAN JOURNAL OF CLINICAL NUTRITION, AMERICAN SOCIETY FOR NUTRITION, US, vol. 85, no. 5, 1 May 2007 (2007-05-01), pages 1185-1196, XP002466268, ISSN: 0002-9165

## Description

### FIELD OF THE INVENTION

The present invention refers to a composition comprising diamine oxidase (DAO) for use in the treatment or prevention of fibromyalgia or chronic fatigue syndrome.

### BACKGROUND OF THE INVENTION

Fibromyalgia (etymologically the term is derived from the Latin and Greek: pain of the muscle of the connective tissue) is defined as a group of symptoms and musculoskeletal disorders that are fundamentally characterised by extreme fatigue, persistent pain, variable amount of stiffness of the muscles, tendons and surrounding soft tissue and a wide range of other psychological symptoms such as sleep difficulties, morning stiffness, headaches and memory problems (the so-called "memory lapses") that often prevent routine functioning of the subject.

Overall prevalence is 2.37 %, affecting mostly women (in a proportion between 8/1 and 20/1), particularly younger women between 20 and 50 years of age.

Diagnosis is controversial owing to the absence of abnormalities found in physical examination, target laboratory examinations or studies of medical images for diagnostic confirmation. Its origin is due to abnormalities in the central nervous system affecting brain regions that may be linked to the clinical symptoms.

The main characteristic of fibromyalgia is diffuse and generalised musculoskeletal pain or prominent stiffness that affects at least 3 anatomical locations for over 3 months,^{[ ]} without which the disorder cannot be diagnosed. The pain is usually intense and often difficult to describe, and in general gets worse with intense physical exercise, cold and emotional stress.

The locations where the symptoms of fibromyalgia often appear are the lumbar region (lower back), neck, thorax and thighs. The disorder of the muscles refers to a painful and localised cramp that is sometimes associated with other problems (for example pregnancy). Sometimes, localised muscle spasm is observed.

Other additional symptoms may include urinary incontinence, headache, migraines, abnormal periodic movements of the extremities (paroxysmal movements), especially of the feet (trigger leg syndrome), difficulty in concentrating and difficulty in remembering things (poor memory); also common is an increase in tactile sensitivity, generalised itching, dry eyes and mouth, hum and ringing in the ears (tinnitus), visual disturbances (phosphene) and some neurological symptoms of lack of motor coordination. Raynaud's phenomenon has been associated as a clinical manifestation occasionally found during the course of this disease.

Between 70 % and 90 % of those suffering from fibromyalgia also report sleep disorders, expressed as a non-refreshing, light and unstable sleep. A heterogeneous group of symptoms are often additionally associated including severe (adynamia) and even incapacitating (asthenia) weakness, disorders of the intestinal rhythm, stiffness in upper or lower extremities and, very frequently, depressive episodes accompanied by panic attacks. Sleep disorders are very common in patients with this pathology. These disorders basically consist of frequent nightmares, non-refreshing sleep, which can be the cause of a disorder known as diurnal hypersomnia, and a high number of painful discharges in the muscles during sleep.

Extreme fatigue is present in all activities carried out by people with fibromyalgia, so everyday tasks are inevitably impeded. Depending on the seriousness and of the variation in degree, this tiredness can range from bearable to being an almost insurmountable disability limiting tasks both at home and at work.

Related to this tiredness, as a causal or aggravating condition, is poor quality of sleep, which prevents those with this disorder from having a refreshing sleep and, consequently, prevents rest, which accentuates tiredness and fatigue in the future.

With respect to treatment, it is thought that there is currently no cure for fibromyalgia and the treatments leading to relief from the symptoms require the intervention of a multidisciplinary team. The treatments used include antidepressives, muscle relaxants, serotonin inhibitors, NSAIDs, etc. In addition, rehabilitation, trigger point (myofascial) massage, craniosacral therapy, chiropractic treatment, osteopathy, stretching exercises and/or aerobics, alternative medicine, posture training, occupational therapy, relaxation therapy, behavioural therapy, nutrition, acupuncture, etc. are considered suitable.

Chronic fatigue syndrome is a disease considered by the WHO as a serious neurological disease that can progressively affect the immune, neurological and cardiovascular and endocrine systems. It is characterised by manifestations including severe fatigue, feverishness or fever, non-refreshing sleep, intolerance to light, sound and temperature changes, muscular pain and pain in the joints, multiple chemical sensitivities, electromagnetic sensitivity, sensitivity to other environmental factors, sensation of permanent flu, chronic faringitis, substantial loss of concentration and memory, spatial disorientation, intolerance to emotional stress and physical activity.

The symptomatology is very variable in terms of severity and temporal presentation, ranging from states of prolonged abnormal fatigue with various flu-like symptoms to a very severe chronic disease with many symptoms that can affect the whole body and put the patient in bed for very long periods of time, even leading to a complete inability to carry out any activity for years.

In severe cases, all the following symptomatology may be present: very profound exhaustion, generalised pain, feeling of weakness at the slightest physical, mental or emotional effort, insomnia, etc.

Currently, it is estimated that the disease affects around 0.5 % of the world population and that the proportion by gender is nine women to one man.

Currently, there is no effective medical treatment, either for fibromyalgia or for chronic fatigue.

Musculoskeletal pain common in people suffering from fibromyalgia and chronic fatigue, as well as other additional symptoms of both diseases, result from dilation of the blood vessels by the effect caused by some vasodilator substances, mainly histamine, in the body and by the accumulation of histamine in muscle tissue. Thus histamine has a direct effect in the various manifestations of pain, common in people suffering from fibromyalgia and chronic fatigue, with a key role in the pain processes.

Histamine [2-(4-imidazolyl)ethylamine] is an important mediator of many biological processes including inflammation, secretion of gastric acid, neuromodulation and the regulation of the immune function. Due to its potent pharmacological activity, even at very low concentration, it is necessary to regulate the synthesis, transport, storage, release and degradation of histamine very carefully in order to avoid undesirable reactions. High concentrations of free histamine in the circulation have been described to lead to undesirable effects such as headache, blocked nose or rhinorrhoea, obstruction of respiratory pathways, tachycardia, gastric and intestinal pains, eyelid swelling, cutaneous erythema, reduction of arterial pressure, bronchospasms, etc.

Histamine is produced by human beings and stored in an inactive form in the metachromatic granules of the mast cells and basophilic leukocytes, where it is available for immediate release. The highest concentrations of histamine are measured in the lungs. After release, histamine becomes an extraordinary powerful mediator of a large number of physiological and pathophysiological processes, frequently by interaction with the cytokines.

Histamine can also enter the human body from the outside as it is generated by microbiological action in the course of processing foods and, consequently, is present in substantial quantities in many fermented foods and drinks such as wine, champagne and a large proportion of alcoholic drinks.

Therefore after ingesting certain foods, a significant increase in circulating histamine is produced. The main route of inactivation of ingested histamine is oxidative deamination of the primary amino group, catalysed by diamine oxidase (DAO) to produce imidazole acetaldehyde.

The main function of DAO is to prevent histamine ingested with food from reaching the blood circulation from the intestine.

In addition to histamine, DAO can degrade other biogenic amines such as, for example, putrescine, spermidine and cadaverine. It has a molecular weight of approximately 182 kDa and a carbohydrate ratio of 11 %. It belongs to the copper-containing amine oxidase class that catalyses oxidative deamination of primary amines to give aldehydes, ammonia and hydrogen peroxide. DAO uses molecular oxygen for the oxidative deamination of histamine to imidazole acetaldehyde, ammonia and hydrogen peroxide.

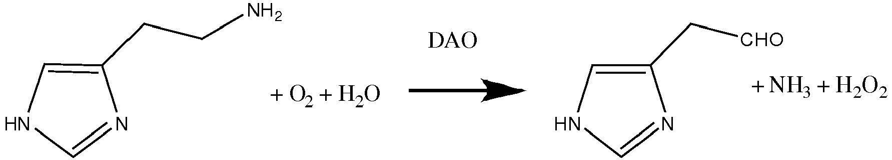

### Histamine Imidazole acetaldehyde

DAO is mainly found in the small intestine, liver, kidneys and blood leukocytes. The level of DAO in the blood of pregnant women is approximately 500 to 1000 times higher than of non-pregnant women as DAO in pregnant women is additionally formed in the placenta. Histamine is continually produced in humans and is excreted via the intestine, being degraded on passing through intestinal mucosa by the DAO found there.

DAO is a sensitive enzyme that can be inhibited by different substances such as other biogenic amines, alcohol and by its degradation product acetaldehyde, as well as by various medicinal drugs.

In addition to inhibition of DAO by certain types of substances, there is a significant percentage of the population in which blood DAO levels are anomalously low, which leads to levels of histamine in blood being at higher levels than considered normal (2-20 micrograms/0.1 L). A series of pathologies appear in these types of subject caused by high levels of blood histamine.

In this situation, preventative administration and treatment of supplementary amounts of DAO contributes to degradation of the excess histamine.

Patent EP 132674 describes a procedure for the enzymatic separation of free amines in foods containing a high amine content such as chocolate, cheese, especially mature, salami, wine and yeast extracts by the use of amine oxidase enzymes, particularly DAO, obtained from *Aspergillus niger,* in the presence of molecular oxygen. The presence of these free amines in certain foods is thought to cause migraines.

Patent US 4725540 describes a procedure for preparing DAO from a microorganism that makes it such as *Candida crusei* or a bacterium that produces lactic acid in a nutrient medium so that the DAO produced is capable of degrading histamine at a pH of between neutral and approximately 4.

Patent application WO 02/43745 from 2001 describes the systematic use of DAO of plant origin for the treatment of diseases mediated by histamine, particularly for the treatment of allergies in general and anaphylactic reactions in particular. There are also pharmaceutical compositions comprising DAO as the active ingredient, including corresponding dosages and administration protocols. The DAO used originates from plants. There is no mention of the possible use of DAO compositions for the prevention and treatment of fibromyalgia or of chronic fatigue syndrome.

Patent application WO 2006003213 of 2005 refers to pharmaceutical compositions for the treatment of histamine-induced diseases comprising DAO of animal origin where the composition is presented in a form protected against gastric acid for oral or peroral administration. The compositions are particularly directed for the treatment of urticaria, atopic dermatitis and scombrotoxic poisoning. This patent application favours the use of DAO of non-plant origin because it is argued that this form has the advantage that allergens present in plants do not have a negative effect on the administration of DAO, as allergens essentially promote the release of endogenous histamine. The DAO used is preferably obtained from pig kidneys or by recombinant techniques. There is no mention of the possible use of DAO compositions for the prevention and treatment of fibromyalgia or of chronic fatigue syndrome. MAINTZ LAURA ET AL: "Histamine and histamine intolerance", THE AMERICAN JOURNAL OF CLINICAL NUTRITION, AMERICAN SOCIETY FOR NUTRITION, US, vol. 85, no. 5, 1 May 2007 (2007-05-01), pages 1185-1196 discloses capsuls containing DAO to supplement the lack of endogeneous human DAO in patients with histamine intolerance.

### Definitions

"DAO" is the abbreviation used for the enzyme diamine oxidase responsible for catalysing the oxidative deamination of the primary amine group of histamine to give imidazole acetaldehyde. It is responsible for the main route of histamine inactivation. "Fibromyalgia" is a group of musculoskeletal symptoms and disorders fundamentally characterised by:
- extreme fatigue
- persistent pain
- stiffness of variable intensity of muscles, tendons and surrounding soft tissue
- a wide range of other psychological symptoms such as difficulty in sleeping, morning stiffness, headaches and memory problems (so-called "memory lapses") that prevent routine functioning of the subject

"Chronic fatigue" is the feeling of intense and prolonged tiredness or exhaustion (fatigue) that is not relieved with rest, the symptoms of which are similar to the majority of most common viral diseases (muscle pains, headache and fatigue). These symptoms may appear in a few hours or days and may last six months or more.

"Non-plant origin" means all those DAO that are not obtained from plants but from animal organisms or from other non-plant organisms. Thus all DAO isolated from living things falls under this definition.

"Plant origin" means all those DAO that are obtained from plant organisms.

"Biotechnological origin" means all those DAO that are recombinantly prepared from cell cultures or in non-vegetable organisms of any type where the DNA for DAO has been isolated.

"Prevention" means avoiding the appearance of symptoms and disorders that involve any type of pain caused by fibromyalgia or chronic fatigue.

"Treatment" means clinical intervention with the intent to change the natural course of fibromyalgia or chronic fatigue and is carried out during the course of the clinical pathology. The desirable effects of treatment include relief from symptoms, reduction of any direct or indirect pathology of the disease, reduction of the speed of progression of the disease, improvement or partial cure of the pathological status and remission or improved prognosis, as well as prevention of recurrence of the disease.

### SUMMARY OF THE INVENTION

The problem solved by the present invention is the treatment and prevention of the symptoms involving any kind of pain and of the symptoms and disorders that characterise fibromyalgia or chronic fatigue syndrome.

Until the present invention, the relation between the symptoms of fibromyalgia or chronic fatigue and the accumulation of histamine had not been described. Therefore, no attempt had been made to treat the symptoms by treatment with DAO. The surprising effect of the present invention is that the administration of DAO reduces the concentration of histamine in blood and this leads to a significant improvement of the symptoms and disorders characterising fibromyalgia and chronic fatigue syndrome.

The first aspect of the present invention, then, is the use of DAO in the preparation of a composition for the prevention or treatment of the symptoms and disorders caused by fibromyalgia or chronic fatigue or, alternatively, a composition comprising DAO for use in the prevention or treatment of the symptoms and disorders caused by fibromyalgia or chronic fatigue.

The second aspect of the present invention is the use of DAO associated with caffeine for the preparation of a composition for the prevention or the treatment of the symptoms and disorders caused by fibromyalgia or chronic fatigue, particularly of the symptoms of fibromyalgia derived by the arterial vasodilation produced by histamine, or alternatively, the composition comprising DAO further comprising caffeine for use in the prevention or the treatment of the symptoms and disorders caused by fibromyalgia or chronic fatigue, particularly of the symptoms of fibromyalgia derived by the arterial vasodilation produced by histamine.

The third aspect of the present invention is oral DAO formulations, optionally containing caffeine, in the form of tablets, capsules and sachets.

The fourth aspect of the present invention are oral formulations of DAO prepared from free DAO, in powder, lyophilised powder, microcapsules, nanocapsules or liposomes containing DAO and optionally caffeine.
The fifth aspect of the present invention are oral formulations of DAO prepared from free DAO, in powder, lyophilised powder, microcapsules, nanocapsules or gastro-protected liposomes containing DAO and optionally also caffeine.

### DETAILED DESCRIPTION OF THE INVENTION

A first aspect is the use of oral compositions comprising DAO for the preparation of a composition for the prevention or treatment of the symptoms and disorders caused by fibromyalgia or chronic fatigue syndrome.

The DAO used in the present invention can be either of biotechnological origin or extracted from animals or plants.

If the DAO used is of non-plant origin, it is preferably in the form of a lyophilised powder. If the DAO used is of plant origin, it may also be in liquid form.

The different compositions comprising DAO to be used in the preparation of a composition for the prevention or treatment of symptoms or disorders caused by fibromyalgia or chronic fatigue syndrome are in the form of tablets, capsules or sachets containing free DAO in powder, lyophilised powder, microcapsules, nanocapsules or liposomes of DAO with gastric protection.

The different compositions comprising DAO may also contain caffeine to potentiate the effects of prevention and treatment of the symptoms and disorders caused by fibromyalgia or chronic fatigue syndrome.

Caffeine is an alkaloid of the xanthine group that has vasoconstriction capacity. Histamine produces vasodilation and this vasodilation produces pain. Caffeine contributes to vasoconstriction and therefore to alleviate the pain.

The DAO content in compositions of the present invention is between 0.1 and 50 mg per dose, preferably between 2 and 20 mg.

The caffeine content in the compositions of the present invention is between 1 and 100 mg per dose, preferably between 5 and 50 mg.

Compositions comprising DAO for use in the prevention and treatment of the symptoms and disorders caused by fibromyalgia or chronic fatigue syndrome can be taken before, after or with meals.

The use of compositions comprising DAO of the present invention directly affects the histamine level in blood and therefore the symptoms and disorders caused by fibromyalgia or chronic fatigue syndrome as a consequence of accumulated histamine levels.

The compositions of the present invention are prepared from free DAO, in powder, lyophilised powder, microcapsules, nanocapsules or liposomes of DAO that have an enteric coating layer that protects the DAO from gastric acidity, so that the different forms can be packaged directly in sachets or put into a capsule or compressed to give tablets. The enteric coating layer that coats the different forms rapidly disintegrates or dissolves in a neutral or alkaline medium.

In the case of microgranules, the cores can be inert cores based on sugar or similar base over which the DAO is applied or these cores can already contain DAO mixed with other excipients. These excipients can be binders, surfactants, filling materials, disintegrants, alkaline additives or other pharmaceutically acceptable ingredients either alone or in a mixture. The binders can be cellulose in type such as hydroxypropyl methylcellulose, hydroxypropyl cellulose and sodium carboxymethyl cellulose, polyvinylpyrrolidone, sugars, starches and other pharmaceutically acceptable substances with cohesive properties. Suitable surfactants can be from the groups of ionic or non-ionic acceptable surfactants such as, for example, sodium lauryl sulphate.

Alternatively, DAO can be mixed with alkaline compounds and additionally mixed with constituents suitable for formulation in a core material. These core materials can be produced by extrusion/spheronization or by compression using different processing equipment.

DAO can also be mixed with other pharmaceutically acceptable alkaline substances such as salts of phosphoric acid and sodium, potassium, calcium, magnesium and aluminium, carbonic acid, citric acid or other suitably weak organic or inorganic acids; a co-precipitate of aluminium hydroxide/sodium bicarbonate; substances normally used in anti-acid preparations such as aluminium, calcium and magnesium hydroxides; magnesium oxide or compound substances such as Al₂O₃.6MgO.CO₂.12H₂O, (Mg₆Al₂(OH)₁₆CO₃.4H₂O, MgO.Al₂O₃.2SiO₂.nH₂O or similar compounds; pH buffering substances such as tris(hydroxymethyl)aminomethane, basic amino acids and their salts or other pharmaceutically acceptable pH buffering substances.

The enteric coating layers can contain pharmaceutically acceptable plasticizers to obtain the desired mechanical, flexibility and hardness properties. These plasticizers can be, for example, triacetin, citric acid esters, phthalic acid esters, cetyl alcohol, polyethylene glycols, polysorbates or other plasticizers.

The present invention also refers to a composition that comprises DAO according to any of the embodiments of the present invention for use in the prevention or treatment of the symptoms and disorders caused by fibromyalgia or chronic fatigue syndrome.

The present invention also refers to a method of treatment that comprises administrating a composition comprising DAO, according to any of the embodiments of the present invention, to a patient who presents symptoms and disorders caused by fibromyalgia or chronic fatigue syndrome or who is at risk from suffering from them, in a therapeutically effective amount.

### EXAMPLES

### Example 1

Tablets of DAO were prepared from microgranules containing 4 % DAO in accordance with the following formula:

| | |
|---|---|
| DAO | 4 mg |
| Mannitol | 40 mg |
| Microcrystalline cellulose | 25 mg |
| Hydroxypropyl cellulose | 10 mg |
| Maize starch | 10 mg |
| Citric acid | 6 mg |

The microgranules were coated with hydroxypropyl methylcellulose.
To make the tablets, the DAO microgranules were compressed with microcrystalline cellulose and sodium stearyl fumarate.

### Example 2

Tablets of DAO were prepared from microgranules containing 4 % DAO and 10 % caffeine in accordance with the following formula:

| | |
|---|---|
| DAO | 4 mg |
| Caffeine | 10 mg |
| Mannitol | 35 mg |
| Microcrystalline cellulose | 15 mg |
| Hydroxypropyl cellulose | 10 mg |
| Hydroxypropyl methylcellulose | 10 mg |
| Ascorbic acid | 6 mg |

The microgranules were coated with a methacrylic acid copolymer.

To make the tablets, the DAO microgranules were compressed with microcrystalline cellulose and magnesium stearate.

### Example 3

DAO sachets were prepared containing 100 or 150 mg of DAO microgranules prepared according to the first part of example 1.

### Example 4

DAO and caffeine sachets were prepared containing 100 or 150 mg of DAO microgranules prepared according to the first part of example 2.

### Example 5

DAO capsules were prepared containing 100 or 150 mg of DAO microgranules prepared according to the first part of example 1, filling the soft gelatine capsules with the microgranules.

### Example 6

DAO and caffeine capsules were prepared containing 100 or 150 mg of DAO microgranules prepared according to the first part of example 2, filling the soft gelatine capsules with the microgranules.

### Example 7

Determination of the efficacy of DAO compositions, which are an object of the present invention, in subjects diagnosed with fibromyalgia and who showed a deficit of DAO.

Oral compositions containing DAO, alone or associated with caffeine, which are an object of the present invention, were tested in a total of 65 subjects diagnosed with fibromyalgia, as out-patients.

Before assigning the subjects to the study, levels of histamine in blood were measured as well as the activity level of DAO in plasma. Those subjects with blood histamine values over 20 micrograms per decilitre and levels of DAO activity below 40 HDU/mL were included in the study.

After the first analysis, there were 42 selected subjects (38 women and 4 men, of ages between 21 and 45 years) who were randomly assigned DAO compositions or placebo.

The following tables show the results in relation to the reduction of symptoms and disorders caused by fibromyalgia after the administration of a dose protocol of 4 mg DAO twice a day in 21 subjects diagnosed with fibromyalgia and with DAO deficit in comparison with 21 subjects who were administered placebo.

Table 1: Comparative results of the symptoms and disorders caused by fibromyalgia between subjects taking DAO tablets, of Example 1, and those not taking DAO.

The degree of pain, both in the initial diagnosis of fibromyalgia and in monitoring the group treated with DAO and the placebo group, was determined using the McGill pain scales for measuring pain quality and the visual analogue scale (VAS) for measuring pain intensity.

The McGill pain questionnaire, which enables measurement of pain quality, is a questionnaire where the patient must choose a descriptor for each scale best representing their pain experience, deciding if the pain is throbbing, pulling, sharp, etc. and a second category measuring the emotional component where the patient describes the pain choosing between adjectives such as "tiring", "punishing" or "wretched", etc.

The **Visual Analogue Scale** (VAS) is the most commonly used instrument in clinical studies to evaluate the pain intensity of fibromyalgia. The patient is shown a horizontal or vertical line with the ends marked for the absence of pain and the worst possible or imaginable pain; they are asked to mark a point on the line reflecting their pain, and then the distance in millimetres from the no pain end to the point marked by the patient is measured.

"Pain maps" were also used to determine the location and spatial extension of the symptom; however, the maps are only an aid and do not replace a good clinical evaluation. Normally, a map is used with sensitive points for fibromyalgia, where serial evaluation of whether the pain reduces or increases can be evaluated in successive measurements in each of these points. These maps are very useful for monitoring pain.

Pain measurement tests were also based on tolerance to pressure applied at the point affected by the pain using a rubber probe. Patients with fibromyalgia require much less pressure to activate the neurones associated with acute pain in the brain than healthy patients. The magnitude of pain, sensitivity to pain and symptoms of depression were compared in the subjects using an exploratory scanner (MRI).

| **Symptoms and disorders of fibromyalgia** | **Subjects taking DAO: 21** | **Subjects not taking DAO: 21** |
|---|---|---|
| Back pain | 3 of 21 | 20 of 21 |
| Lumbar pain | 4 of 21 | 19 of 21 |
| Neck pain | 4 of 21 | 20 of 21 |
| Knee pain | 5 of 21 | 21 of 21 |
| Fatigue | 5 of 21 | 21 of 21 |
| Histamine level in blood | 2-20 micrograms/0.1 L | >> 20 micrograms/0.1 L |

Table 2: Comparative results of the symptoms and disorders caused by fibromyalgia between subjects taking DAO and caffeine tablets, of Example 2, and those not taking DAO and caffeine. Subjects selected for this trial were diagnosed with fibromyalgia but without associated sleep disorders.

| **Symptoms and disorders of fibromyalgia** | **Subjects taking DAO and caffeine: 21** | **Subjects not taking DAO and caffeine: 21** |
|---|---|---|
| Cephalea | 2 of 21 | 20 of 21 |
| Fatigue | 4 of 21 | 21 of 21 |
| Histamine level in blood | 2-20 micrograms/0.1 L | >> 20 micrograms/0.1 L |

### Example 8

Determination of the efficacy of DAO compositions of the present invention in subjects diagnosed with chronic fatigue syndrome and who showed a deficit of DAO. Oral compositions containing DAO of the present invention were tested in a total of 46 subjects diagnosed with chronic fatigue syndrome, as out-patients.

To evaluate the degree of affectation of chronic fatigue syndrome in candidate study subjects, the quality of life of these subjects was evaluated using a simple scale of 4 successive grades (I, II, III and IV), with criteria used in the clinic. This scale is used in the Chronic Fatigue Syndrome (CFS) Functional Unit in the Hospital Clinic.

The CLINIC scale consists of dividing the patients with chronic fatigue syndrome into four different grades of functional affectation depending on the effect of their fatigue on quality of life:
GRADE I: the patient is sometimes or occasionally fatigued, without significant limitation (< 50 %) of work or daily life activities.
GRADE II: persistent presence of fatigue, oscillating but without improvement, with marked effect (> 50 %) on work and also daily life activities.
GRADE III: marked fatigue that does not allow, even occasionally, performing any type of work, which limits autonomy and activities of daily life by more than 80 %.
GRADE IV: extreme fatigue that requires the help of other people for basic personal activities and that makes autonomy and the activities of daily life impossible.

Improvement is evaluated when the patient passes from a higher to a lower grade in terms of the number of improvement grades.

The following table shows the results in relation to the number of grades of improvement on the CLINIC scale in 23 subjects diagnosed with chronic fatigue syndrome of grades II, III and IV and with DAO deficit, after the administration of a dose protocol of 4 mg DAO twice a day in comparison with 23 subjects administered placebo.

| **Number of grades of improvement** | **Subjects taking DAO: 23** | **Subjects not taking DAO: 23** |
|---|---|---|
| 1 | 16 of 23 | 2 of 23 |
| 2 | 4 of 23 | 0 of 23 |
| Histamine level in blood | 2-20 micrograms/0.1 L | >> 20 micrograms/0.1 L |

## Claims

1. Composition comprising diamine oxidase (DAO) for use in the prevention or treatment of the symptoms and disorders caused by fibromyalgia or chronic fatigue syndrome.

2. Composition for use in accordance with claim 1, **characterized in that** the DAO is administered orally in the form of tablets, capsules or sachets.

3. Composition for use in accordance with any of claims 1 and 2, **characterized in that** the DAO is administered at a dose of between 0.1 and 50 mg.

4. Composition for use in accordance with any of claims 1 to 3, **characterized in that** the composition also contains caffeine.

5. Composition for use in accordance with claim 4, **characterized in that** the caffeine is administered at a dose of between 1 and 100 mg.

6. Composition for use in accordance with any of claims 1 to 5, **characterized in that** the dose forms have gastric protection.

7. Composition for use in accordance with any of claims 1 to 6, **characterized in that** the DAO is used in free form, in powder, lyophilised powder, microcapsules, nanocapsules or liposomes.

## Patentansprüche

1. Zusammensetzung, die Diaminoxidase (DAO) aufweist, für die Verwendung bei der Verhinderung oder Behandlung von Symptomen und Beschwerden, die durch Fibromyalgie oder chronisches Erschöpfungssyndrom verursacht werden.

2. Zusammensetzung für die Verwendung gemäß Anspruch 1, **dadurch gekennzeichnet, dass** das DAO oral verabreicht wird in der Form von Tabletten, Kapseln oder Tütchen.

3. Zusammensetzung für die Verwendung gemäß einem der Ansprüche 1 und 2, **dadurch gekennzeichnet, dass** das DAO mit einer Dosis von zwischen 0,1 und 50 mg verabreicht wird.

4. Zusammensetzung für die Verwendung gemäß einem der Ansprüche 1 bis 3, **dadurch gekennzeichnet, dass** die Zusammensetzung auch Koffein enthält.

5. Zusammensetzung für die Verwendung gemäß Anspruch 4, **dadurch gekennzeichnet, dass** das Koffein mit einer Dosis von zwischen 1 und 100 mg verabreicht wird.

6. Zusammensetzung für die Verwendung gemäß einem der Ansprüche 1 bis 5, **dadurch gekennzeichnet, dass** die Dosisformen einen Magenschutz aufweisen.

7. Zusammensetzung für die Verwendung gemäß einem der Ansprüche 1 bis 6, **dadurch gekennzeichnet, dass** das DAO in freier Form, in Pulver, lyophilisiertem Pulver, Mikrokapseln, Nanokapseln oder Liposomen verwendet wird.

## Revendications

1. Composition comprenant une diamine oxydase (DAO) pour utilisation dans la prévention ou le traitement des symptômes et des troubles causés par la fibromyalgie ou le syndrome de fatigue chronique.

2. Composition pour utilisation selon la revendication 1, **caractérisée en ce que** la DAO est administrée par voie orale sous la forme de comprimés, de capsules ou de sachets.

3. Composition pour utilisation selon l'une quelconque des revendications 1 et 2, **caractérisée en ce que** la DAO est administrée à une dose entre 0,1 et 50 mg.

4. Composition pour utilisation selon l'une quelconque des revendications 1 à 3, **caractérisée en ce que** la composition contient également de la caféine.

5. Composition pour utilisation selon la revendication 4, **caractérisée en ce que** la caféine est administrée à une dose entre 1 et 100 mg.

6. Composition pour utilisation selon l'une quelconque des revendications 1 à 5, **caractérisée en ce que** les formes galéniques présentent une protection gastrique.

7. Composition pour utilisation selon l'une quelconque des revendications 1 à 6, **caractérisée en ce que** la DAO est utilisée sous forme libre, de poudre, de poudre lyophilisée, de microcapsules, de nanocapsules ou de liposomes.
